# EUROPEAN PATENT SPECIFICATION

(11) **EP 2 201 020 B1**
(45) Date of publication and mention of the grant of the patent: **16.07.2014**
(21) Application number: 08805203.0
(22) Date of filing: 10.10.2008
(51) Int. Cl.: C07H 19/12

(54) **METHOD OF PRODUCING 2' -DEOXY-5-AZACYTIDINE (DECITABINE)**
VERFAHREN ZUR HERSTELLUNG VON 2'-DEOXY-5-AZACYTIDINE (DECITABINE)
PROCÉDÉ DE PRÉPARATION DE 2'-DEOXY-5-AZACYTIDINE (DECITABINE)

(30) Priority: 10.10.2007 EP 07019826
(43) Date of publication of application: 30.06.2010
(73) Proprietor: Cilag AG, 8205 Schaffhausen (CH)
(72) Inventor: JUNGMANN, Oliver, 78166 Donaueschingen (DE); KRAUT, Norbert, 78250 Tengen (DE)
(74) Representative: Verberckmoes, Filip Gerard
(86) International application number: PCT/EP2008/063581
(87) International publication number: WO 2009/047313

(56) References cited:
- US-A- 3 817 980
- US-A- 4 082 911
- US-A- 4 209 613
- US-A- 5 811 540
- BEN-HATTAR, JEAN ET AL: "An improved synthesis of 2'-deoxy-5-azacytidine by condensation of an 9-fluorenylmethoxycarbonyl-protected sugar onto the silylated base", JOURNAL OF ORGANIC CHEMISTRY , 51(16), 3211-13 CODEN: JOCEAH; ISSN: 0022-3263, 1986, XP002484923,
- LU K-C ET AL: "Simple and efficient per-O-acetylation of carbohydrates by lithium perchlorate catalyst", TETRAHEDRON, ELSEVIER SCIENCE PUBLISHERS, AMSTERDAM, NL, vol. 60, no. 40, 27 September 2004 (2004-09-27), pages 8967-8973, XP004565047, ISSN: 0040-4020, DOI: 10.1016/J.TET.2004.06.138
- WINKLEY, MICHAEL W. ET AL: "Direct glycosylation of 1,3,5-triazinones. New approach to the synthesis of the nucleoside antibiotic 5-azacytidine (4-amino-1-.beta.-D- ribofuranosyl-1,3,5-triazin-2-one) and related derivatives", JOURNAL OF ORGANIC CHEMISTRY , 35(2), 491-5 CODEN: JOCEAH; ISSN: 0022-3263, 1970, XP002484924,

## Description

The present invention refers to a method of producing 2'-deoxy-5-azacytidine (Decitabine) by reacting a glycoside donor preferably a 1-halogen derivative, or an imidate preferably a trichloromethyl derivative, or a thio-alkyl derivative of a blocked monosaccharide with a selected silylated base in the presence of a selected catalyst.

### State of the Art

Decitabine is a nucleoside and a known pharmaceutically active compound. From US 3,817,980 it is known to synthesize nucleosides by silylating a corresponding nucleoside base and reacting the silylated base with a glycosyl donor preferably a 1-halogen derivative of a blocked monosaccharide in the presence of a selected catalyst. The catalysts used are e.g. selected from SnCl₄, TiCl₄, ZnCl₂, BF₃-etherate, AlCl₃ and SbCl₅. The major disadvantage is that these catalysts are prone to hydrolysis giving irritant hydrolysis products like HCl and/or are forming insoluble oxides (TiO₂, SnO₂), which are difficult to remove from the reaction product. These catalysts are difficult to handle, especially on large scale production.

US-A-4 082 911 refers to the analogous process of reacting a silylated nucleoside base with a protected derivative of a sugar and proposes to use as catalyst a trialkylsilyl ester of a strong organic acid, such as trimethylsilyl-trifluoromethanesulfonate. US-A-4 209 613 proposes an improvement for the method disclosed in US-A-4 082 911 by using a single-step process wherein the trialkylsilyl ester of the strong organic acid, such as trimethylsilyl-trifluoromethanesulfonate, is formed in situ from the free acid by reaction of the free acid with the silylating agent, e.g. trialkylchlorosilane, which is present in the appropriate molar amount. Silylating agents such as trialkylchlorosilane, are very reactive and quickly react to form the trialkylsilyl ester of the free acid present in the reaction mixture. Ben-Hatter Jean et al., J. Org. Chem., vol. 51, p. 3211 - 3213 (1986), discloses the preparation of decitabine by reacting a silylated nucleoside base with a protected nucleoside sugar using the Friedel-Craft catalyst SnCl₄.

### Description of the invention

It has now been found that a 1-halo monosaccharide derivative can be reacted with a silylated or alkylated 5-azacytosine in the presence of a salt as a catalyst wherein said catalyst is selected from the group comprising a salt of an aliphatic sulphonic acid such as trifluoromethane sulfonate. There is no need to use an ester compound as a catalyst. This very much simplifies the production of 2'-deoxy-5-azacytidine (Decitabine) as described in the present invention. Furthermore, using the catalyst of the present invention an improved selectivity in favor of the beta-isomer (β-isomer) may be obtained, e.g. a selectivity of at least 1:2. The reaction of the present invention can be carried out so that about three quarters of the reaction yield is the beta isomer and, depending on the particular reaction conditions, a ratio of the alpha to the beta isomer of 12:88 was obtained. Further, according to the present invention a reaction yield that is higher than 95%, and regularly is within the range of 97-99%, calculated to the total amount of anomers present in the final crude reaction mixture, can be obtained.

The type of catalyst as used according to the present invention is stable under aqueous conditions, easy to handle, does not produce irritant hydrolysis products, and can be easily removed. Additionally, the selectivity of the reaction for obtaining the desired anomer, i.e. the ratio of the alpha/beta anomers, and the final yields are considerably improved.

The present invention is defined in the claims. The present invention refers to a method of producing 2'-deoxy-5-azacytidine (Decitabine) by providing a compound (a blocked monosaccharide derivative) of formula (I): 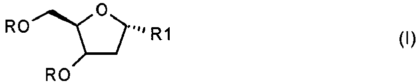 wherein
R is a removable substituent (protecting group) known per se, preferably (C₁-C₈)alkylcarbonyl, or optionally substituted phenylcarbonyl, or optionally substituted benzylcarbonyl;
R₁ is a removable substituent preferably halogen, preferably chlorine, bromine, fluorine, preferably chlorine, or an imidate, preferably trichloromethyl imidate, or a thio-alkyl derivative, preferably -S-methyl;
further providing a silylated base of formula (II): 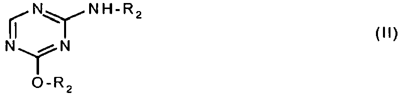 wherein R₂ is a protecting group, preferably a trimethylsilyl (TMS)-residue;
reacting the compound of formula (I) and the compound of formula (II) together in a suitable anhydrous solvent and in the presence of a suitable catalyst, whereby the compound of formula (III): 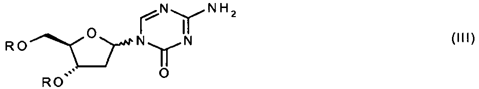 is obtained; and removing the substituent R in order to obtain the compound 2'-deoxy-5-azacytidine (Decitabine), characterized in that said catalyst is a salt of an aliphatic sulphonic acid.

The present invention refers also to the production of the compound of formula (III) using a catalyst of the present invention, yielding a desired selectivity, preferably in favor of the beta-isomer (β-isomer), preferably at a ratio of at least 1:2, and preferably wherein about three quarters of the reaction yield is the beta isomer. Preferred is the beta-glycoside of formula (III).

The catalyst used in said reaction is a salt of an aliphatic sulphonic acid, said catalyst preferably is a salt of methylsulphonic acid (mesylate) or of ethylsulphonic acid, or is a salt of a fluorinated aliphatic sulfonic acid, such as a salt of trifluoromethane-sulfonic acid, of pentafluoroethyl-sulfonic acid, or of heptafluoropropyl-sulfonic acid.

Preferred of these salts are the salts of methylsulphonic acid (mesylate), and the salts of trifluoromethanesulfonic acid.

Preferred aliphatic sulphonic acid salts and fluorinated aliphatic sulfonic acid salts are the alkali salts and earth alkali salts, preferably the salts of lithium, sodium, potassium, or magnesium. Preferred are the lithium salts, preferably lithium methylsulphonic acid (lithium mesylate), lithium-trifluoromethanesulfonate (LiOTf, lithiumtriflate), and lithium tetrafluoroborate. Also other salts, for example the salts of scandium, such as Sc(OTf)₃, of zinc such as Zn(OTf)₂, or of copper such as Cu(OTf)₂ can be used. However, the lithium salt and especially LiOTf is preferred.

Preferred solvents to carry out the reaction according to the present invention are organic solvents such as benzene, toluene, xylol, or chlorinated solvents, for example dichloromethane, dichloroethane, chloroform, chlorobenzene, or acetonitril and/or propylene carbonate and/or related solvents. Preferred are toluene and chlorinated solvents. Preferred is the use of lithium-trifluoromethanesulfonate (LiOTf) in a chlorinated solvent, preferably in dichloromethane, dichloroethane, chloroform, chlorobenzene and/or in an aromatic solvent like toluene or xylene. Each solvent or mixture of solvents may yield a different selectivity with respect to the beta-isomer (β-isomer). It is no problem for the expert in the art to optimize the catalyst and/or solvent or the mixture of solvents in order to obtain the desired selectivity in favor of the beta-isomer.

The compound of formula (I) is a glycoside donor compound. The preparation of the compound of formula (I) is known per se.

The removable substituent R is preferably (C₁-C₄)alkylcarbonyl, or optionally substituted phenylcarbonyl, like phenylcarbonyl, tolylcarbonyl, xylylcarbonyl or benzylcarbonyl; preferably acetyl or p-chloro-phenylcarbonyl.

The removable substituent R₁ is preferably halogen, preferably chlorine, bromine, fluorine, preferably chlorine, or an imidate, preferably trichloromethyl imidate [-NH-(O)C-CCl₃], or a thio-alkyl derivative, preferably -S-methyl.

The compound of formula (II) and its preparation are known. The compound is preferably prepared by reaction of the free base with trimethylchlorosilane or with hexamethyldisilazane.

When reacting the compounds of formulae (I) and (II) together, the reaction temperature generally is within the range of 0°C to about 90°C, preferably at about room temperature, whereby the components are reacted in about equimolar amounts or with an excess of compound of formula (II). The catalyst is used preferably in a concentration of about 10 mol-% to 100 mol-%, calculated to the total molar presence of the two reacting components. For the expert in the art it is no problem to optimize the molar ratios of the components.

For removing the substituents R from the compound of formula (III) in order to obtain the compound 2'-deoxy-5-azacytidine (Decitabine), containing free hydroxyl groups, known methods are used. The substituents R may be preferably removed, for example, by treatment in an alcoholic solution of ammonia or alcoholates; but other known methods may be applied. The following example illustrates the invention.
Example 1
   (A) A mixture of 5-azacytosine (20 g, 178.4 mmol), ammonium sulfate (2.4 g, 18.16 mmol), and hexamethyldisilazane (160 g, 991.3 mmol) was heated to reflux until a clear solution was obtained. The excess of hexamethyldisilazane was removed in the vacuum at 60°C.
   (B) 264 g of dichloromethane, followed by lithium trifluoro-methane sulfonate (27.84 g, 178.4 mmol) and the "chloro sugar" C-137: 1-chloro-3,5-di-o-*p*-chlorobenzoyl-2-deoxy-α-D-ribofuranose [76.67 g, 178.4 mmol, corresponding to compound of formula (I)] were added to the residue obtained in step (A).
   (C) The mixture was stirred for 4 hours at ambient temperature (20-25°C). Reaction yield combined anomers 99.2%, selectivity alpha/beta 27/73.
   (D) Then the solvent was removed at 40°C in the vacuum and the obtained residue was dissolved in 60 g ethyl acetate. The solution was added dropwise to a mixture of 220 g of aqueous sodium hydrogen carbonate (2.5% w-solution), 174 g ethyl acetate, 36 g cyclohexane and 70 g acetonitrile at 30°C and the obtained reaction mixture is cooled to 0°C and stirred for 3 hours (h). The precipitate of the blocked (protected) aminotriazine was filtered off, washed with water and finally with a mixture of acetonitrile and ethyl acetate (1:1).
      Total yield 79.2 g (87.8%) combined anomers; ratio alpha/beta 31:69. Scheme 1 shows the chemical reaction.
Example 2: The compound corresponding to formula (III) as obtained in Example 1 is further treated with in an alcoholic solution of ammonia in a known manner so that 2'-deoxy-5-aza-cytidine (Decitabine) is obtained in practically quantitative yield. 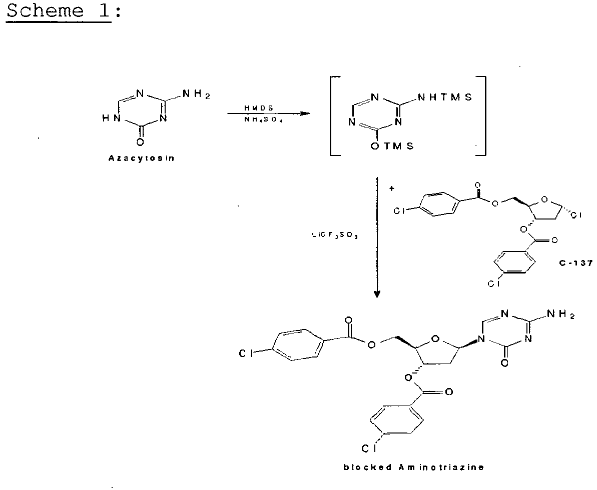
Example 3: Example 1 was repeated using 1.0 equivalents of lithium mesylate instead of lithium trifluoromethane sulfonate. Reaction yield after step (C): Combined anomers 95.2%, selectivity alpha/beta 60:40.
   Total yield after work-up step (D) 85.2% combined anomers; ratio alpha/beta 63:37.
Reference Example 4: Example 1 was repeated using 1.0 equivalents of lithium perchlorate instead of lithium trifluoromethane sulfonate.
   Reaction yield after step (C): Combined anomers 99.4%, selectivity alpha/beta 37:63.
   Total yield after work-up step (D) 85.2% combined anomers; ratio alpha/beta 36:64.
Example 5: Example 1 was repeated using 1.0 equivalents of lithium tetrafluoroborate instead of lithium trifluoromethane sulfonate.
   Reaction yield after step (C): Combined anomers 94.5%, selectivity alpha/beta 59:41.
   Total yield after work-up step (D) 47.9% combined anomers; ratio alpha/beta 70:30.
Example 6: Example 1 was repeated using 1.0 equivalents of sodium trifluoromethane sulfonate instead of lithium trifluoromethane sulfonate.
   Reaction yield after step (C): Combined anomers 99.2%, selectivity alpha/beta 40:60.
   Total yield after work-up step (D) 80.7% combined anomers; ratio alpha/beta 40:60.
Example 7: Example 1 was repeated using 1.0 equivalents of potassium trifluoromethane sulfonate instead of lithium trifluoromethane sulfonate.
   Reaction yield after step (C): Combined anomers 99.0%, selectivity alpha/beta 44:56.
   Total yield after work-up step (D) 79.9% combined anomers; ratio alpha/beta 46:54.
Example 8: Example 1 was repeated [except for step (D)] using 1.0 equivalent of zinc trifluoromethane sulfonate instead of lithium trifluoromethane sulfonate.
   Reaction yield after step (C): Combined anomers 96.0%, selectivity alpha/beta 54:46.
Example 9: Example 1 was repeated using the same volume of toluene instead of dichloromethane as solvent.
   Reaction yield after step (C): Combined anomers 99.4%, selectivity alpha/beta 27:73.
   Total yield after work-up step (D) 88.7% combined anomers; ratio alpha/beta 31:69.
Example 10: Example 1 was repeated using the same volume of acetonitrile instead of dichloromethane as solvent.
   Reaction yield after step (C): Combined anomers 99.2%, selectivity alpha/beta 50:50.
   Total yield after work-up step (D) 82.5% combined anomers; ratio alpha/beta 52:48.
Example 11
   (A) A mixture of 5-azacytosine (0.5 g, 4.46 mmol, 1 equ.), ammonium sulfate (40 mg, 0.3 mmol, 0.07 equ.), and hexamethyl-disilazane (4 g, 24.8 mmol, 5.6 equ.) was heated to reflux until a clear solution was obtained. The excess of hexamethyldisilazane was removed in the vacuum at 60°C.
   (B) Afterwards 10 ml of dichloromethane, lithium trifluoromethane-sulfonate (0.33 g, 2.11 mmol; 0.47 equ.) and the "chloro sugar" C-137: 1-Chloro-3,5-di-0-p-chlorobenzoyl-2-deoxy-alpha-D-ribofuranose [0.73 g, 1.70 mmol, 0.38 equ.; corresponding to compound of formula (I)] were added to the residue obtained in step (A). The mixture was stirred for 4 hours at ambient temperature (20-25°C).
      Reaction yield combined anomers 99.1%; alpha/beta = 16/84.
Example 12: Example 11 was repeated using 0.47 equivalents of copper trifluoromethane sulfonate instead of lithium trifluoromethane sulfonate.
   Reaction yield after step (B): Combined anomers 98.0%, selectivity alpha/beta 42:58.
Example 13: Example 11 was repeated using 0.47 equivalents of scandium trifluoromethane sulfonate instead of lithium trifluoromethane sulfonate.
   Reaction yield after step (B): Combined anomers 88.0%, selectivity alpha/beta 43:57.
Example 14: Example 11 was repeated using 0.47 equivalents of magnesium trifluoromethane sulfonate instead of lithium trifluoromethane sulfonate.
   Reaction yield after step (B): Combined anomers 89.0%, selectivity alpha/beta 58:42.
Example 15: Example 11 was repeated using the same volume of acetonitrile instead of dichloromethane as solvent.
   Reaction yield after step (B): Combined anomers 97.6%, selectivity alpha/beta 39:61.
Example 16: Example 11 was repeated using the same volume of chlorobenzene instead of dichloromethane as solvent.
   Reaction yield after step (B): Combined anomers 96.2%, selectivity alpha/beta 26:74.
Example 17: Example 11 was repeated using the same volume of propylencarbonate instead of dichloromethane as solvent.
   Reaction yield after step (B): Combined anomers 96.8%, selectivity alpha/beta 42:58.
Example 18: Example 11 was repeated a mixture of 10 ml of dichloromethane and 3.5 ml of xylene instead of 10 ml of pure dichloromethane as solvent.
   Reaction yield after step (B): Combined anomers 93.3%, selectivity alpha/beta 27:73.
Example 19
   (A) A mixture of 5-azacytosine (0.5 g, 4.46 mmol, 1 equ.), ammonium sulfate (40 mg, 0.3 mmol, 0.07 equ.), and hexamethyl-disilazane (4 g, 24.8 mmol, 5.6 equ.) was heated to reflux until a clear solution was obtained.
   (B) Afterwards 10 ml of 1,2-dichlorobenzene, lithium trifluoromethane-sulfonate (0.33 g, 2.11 mmol; 0.47 equ.) and the "chloro sugar" C-137: 1-Chloro-3,5-di-O-p-chlorobenzoyl-2-deoxy-alpha-D-ribofuranose; [1.15 g, 2.68 mmol, 0.60 equ.; corresponding to compound of formula (I)] were added to the residue obtained in step (A). The mixture was stirred for 4 hours at ambient temperature (20-25°C).
      Reaction yield combined anomers 91.2%; alpha/beta = 27/73.
Example 20: Example 19 was repeated using the same volume of 1,2-dichloroethane instead of 1,2-dichlorobenzene as solvent. Reaction yield after step (B): Combined anomers 93.4%, selectivity alpha/beta 27:73.
Example 21
   (A) A mixture of 5-azacytosine (0.5 g, 4.46 mmol, 1 equ.), ammonium sulfate (40 mg, 0.3 mmol, 0.07 equ.), and hexamethyl-disilazane (4 g, 24.8 mmol, 5.6 equ.) was heated to reflux until a clear solution was obtained. The excess of hexamethyldisilazane was removed in the vacuum at 60°C.
   (B) Afterwards 10 ml of dichloromethane, lithium trifluoromethanesulfonate (0.33 g, 2.11 mmol; 0.47 equ.) and the "chloro sugar" C-137: 1-Chloro-3,5-di-O-p-chlorobenzoyl-2-deoxy-alpha-D-ribofuranose; [0.38 g, 0.88 mmol, 0.20 equ.; corresponding to compound of formula (I)] were added to the residue obtained in step (A). The mixture was stirred for 4 hours at ambient temperature (20-25°C).
      Reaction yield combined anomers 99.3%; alpha/beta = 12/88.

## Claims

1. Method of producing 2'-deoxy-5-azacytidine (Decitabine) by providing a compound of formula (I): 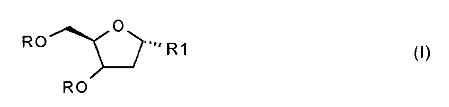 wherein
R is a removable substituent known per se, preferably (C₁-C₈)alkylcarbonyl, or optionally substituted phenylcarbonyl, or optionally substituted benzylcarbonyl;
R₁ is a removable substituent preferably halogen, preferably chlorine, bromine, fluorine, preferably chlorine, or an imidate, preferably trichloromethyl imidate, or a thio-alkyl derivative, preferably -S-methyl;
further providing a silylated base of formula (II): 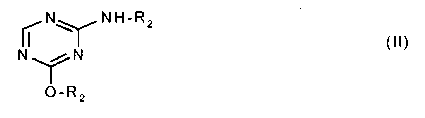 wherein R₂ is a protecting group, preferably a trimethylsilyl (TMS)-residue;
reacting the compound of formula (I) and the compound of formula (II) together in a suitable anhydrous solvent and in the presence of a suitable catalyst, whereby the compound of formula (III): 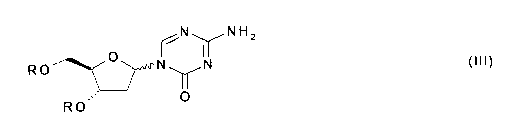 is obtained; and removing the substituents R in order to obtain the compound 2'-deoxy-5-azacytidine (Decitabine), **characterized in that** said catalyst is a salt of an aliphatic sulphonic acid or a salt of a fluorinated aliphatic sulfonic acid.

2. Method of producing a compound of formula (III) according to claim 1, by providing a compound of formula (I): 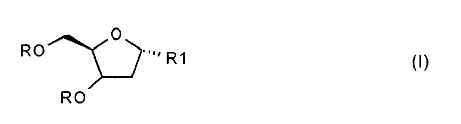 wherein
R is a removable substituent known per se, preferably (C₁-C₈)alkylcarbonyl, or optionally substituted phenylcarbonyl, or optionally substituted benzylcarbonyl;
R₁ is a removable substituent preferably halogen, preferably chlorine, bromine, fluorine, preferably chlorine, or an imidate, preferably trichloromethyl imidate, or a thio-alkyl derivative, preferably -S-methyl;
further providing a silylated base of formula (II): 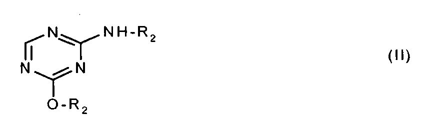 wherein R₂ is a protecting group, preferably a trimethylsilyl (TMS)-residue;
reacting the compound of formula (I) and the compound of formula (II) together in a suitable anhydrous solvent and in the presence of a suitable catalyst, whereby the compound of formula (III) is obtained, **characterized in that** said catalyst is a salt of an aliphatic sulphonic acid or a salt of a fluorinated aliphatic sulphonic acid.

3. Method according to claim 1 or 2, **characterized in that** the catalyst used in said reaction is a salt of methylsulphonic acid or of ethylsulphonic acid, or a salt of trifluoromethane-sulfonic acid, pentafluoroethyl-sulfonic acid, or heptafluoropropyl-sulfonic acid.

4. Method according to claim 3, **characterized in that** the catalyst is a salt of methylsulphonic acid and/or the salts of trifluoromethanesulfonic acid.

5. Method according to any one of the claims 1-4, **characterized in that** the catalyst is an alkali salt or an earth alkali salt, preferably a salt of lithium, sodium, potassium, or magnesium, preferably a lithium salt.

6. Method according to any one of the claims 1-5, **characterized in that** the catalyst is lithium methylsulphonic acid and/or lithium-trifluoromethanesulfonate.

7. Method according to claim 1-4, **characterized in that** the catalyst is chosen from the salts comprising salts of scandium, preferably Sc(OTf)₃, of zinc preferably Zn(OTf)₂, or of copper preferably Cu(OTf)₂.

8. Method according to any one of the claims 1-7, **characterized in that** the solvent to carry out the reaction is chosen from the group comprising organic solvents, preferably benzene, toluene, xylene, or chlorinated solvents, preferably dichloro-methane, dichloroethane, chloroform, chlorobenzene, or toluene, xylol, or acetonitril, propylene carbonate and related solvents,

9. Method according to claim 8, **characterized in that** the solvent to carry out the reaction is chosen from organic solvents, preferably toluene and xylene and chlorinated solvents, preferably from chlorinated solvents.

10. Method according to any one of the claims 1-7, **characterized in that** the catalyst is lithium-trifluoromethanesulfonate and the solvent is chosen from organic solvents, preferably toluene and xylene, and chlorinated solvents, preferably dichloro-methane, dichloroethane, chloroform and/or chlorobenzene.

11. Method according to any one of the claims 1-10, characterrized in that the removable substituent R is (C₁-C₄)alkyl-carbonyl, or optionally substituted phenylcarbonyl or benzylcarbonyl, preferably phenylcarbonyl, tolylcarbonyl, xylylcarbonyl; preferably acetyl or p-chloro-phenylcarbonyl.

12. Method according to any one of the claims 1-11, **characterized in that** the removable substituent R₁ is chlorine.

## Patentansprüche

1. Verfahren zur Herstellung von 2'-Desoxy-5-azacytidin (Decitabin) durch Bereitstellen einer Verbindung der Formel (I): 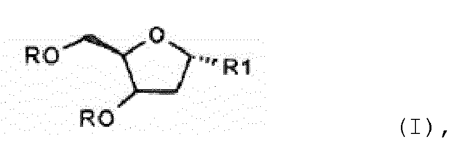 wobei
R für einen an sich bekannten entfernbaren Substituenten, vorzugsweise (C₁-C₈) -Alkylcarbonyl, oder gegebenenfalls substituiertes Phenylcarbonyl, oder gegebenenfalls substituiertes Benzylcarbonyl, steht;
R₁ für einen entfernbaren Substituenten, vorzugsweise Halogen, bevorzugt Chlor, Brom, Fluor, vorzugsweise Chlor, oder ein Imidat, vorzugsweise Trichlormethylimidat, oder ein Thioalkylderivat, vorzugsweise -S-Methyl, steht;
weiterhin Bereitstellen einer silylierten Base der Formel (II) : 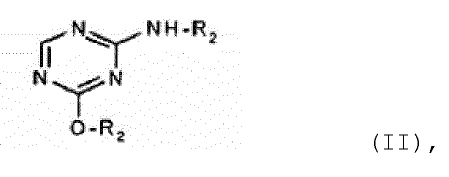 wobei R₂ für eine Schutzgruppe, vorzugsweise einen Trimethylsilyl(TMS)-Rest, steht;
Umsetzen der Verbindung der Formel (I) mit der Verbindung der Formel (II) in einem geeigneten wasserfreiem Lösungsmittel und in Gegenwart eines geeigneten Katalysators, wodurch man die Verbindung der Formel (III) : 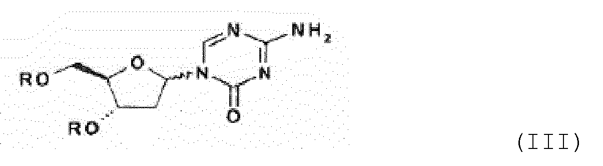 erhält; und Entfernen der Substituenten R unter Erhalt der Verbindung 2'-Desoxy-5-azacytidin (Decitabin),
**dadurch gekennzeichnet, dass** es sich bei dem Katalysator um ein Salz einer aliphatischen Sulfonsäure oder ein Salz einer fluorierten aliphatischen Sulfonsäure handelt.

2. Verfahren zur Herstellung einer Verbindung der Formel (III) nach Anspruch 1 durch Bereitstellung einer Verbindung der Formel (I): 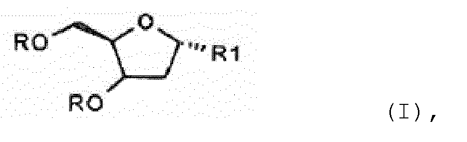 wobei
R für einen an sich bekannten entfernbaren Substituenten, vorzugsweise (C₁-C₈)-Alkylcarbonyl, oder gegebenenfalls substituiertes Phenylcarbonyl, oder gegebenenfalls substituiertes Benzylcarbonyl, steht;
R₁ für einen entfernbaren Substituenten, vorzugsweise Halogen, bevorzugt Chlor, Brom, Fluor, vorzugsweise Chlor, oder ein Imidat, vorzugsweise Trichlormethylimidat, oder ein Thioalkylderivat, vorzugsweise -S-Methyl, steht;
weiterhin Bereitstellen einer silylierten Base der Formel (II) : 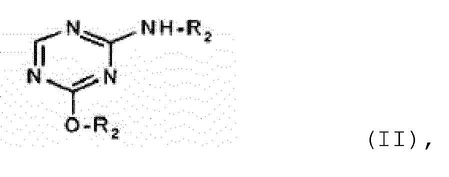 wobei R₂ für eine Schutzgruppe, vorzugsweise einen Trimethylsilyl(TMS)-Rest, steht;
Umsetzen der Verbindung der Formel (I) mit der Verbindung der Formel (II) in einem geeigneten wasserfreiem Lösungsmittel und in Gegenwart eines geeigneten Katalysators, wodurch man die Verbindung der Formel (III) erhält, **dadurch gekennzeichnet, dass** es sich bei dem Katalysator um ein Salz einer aliphatischen Sulfonsäure oder ein Salz einer fluorierten aliphatischen Sulfonsäure handelt.

3. Verfahren nach Anspruch 1 oder 2, **dadurch gekennzeichnet, dass** es sich bei dem in der Umsetzung verwendeten Katalysator um ein Salz von Methylsulfonsäure oder von Ethylsulfonsäure oder ein Salz von Trifluormethansulfonsäure, Pentafluorethylsulfonsäure oder Heptafluorpropylsulfonsäure handelt.

4. Verfahren nach Anspruch 3, **dadurch gekennzeichnet, dass** es sich bei dem Salz um ein Salz von Methylsulfonsäure und/oder die Salze von Trifluormethansulfonsäure handelt.

5. Verfahren nach einem der Ansprüche 1-4, **dadurch gekennzeichnet, dass** es sich bei dem Katalysator um ein Alkalisalz oder ein Erdalkalisalz, vorzugsweise ein Salz von Lithium, Natrium, Kalium oder Magnesium, bevorzugt ein Lithiumsalz, handelt.

6. Verfahren nach einem der Ansprüche 1-5, **dadurch gekennzeichnet, dass** es sich bei dem Katalysator um Lithiummethylsulfonsäure und/oder Lithiumtrifluormethansulfonat handelt.

7. Verfahren nach einem der Ansprüche 1-4, **dadurch gekennzeichnet, dass** der Katalysator aus den die Salze von Scandium, vorzugsweise SC(OTf)₃, von Zink, vorzugsweise Zn(OTf)₂, oder von Kupfer, vorzugsweise Cu(OTf)₂, umfassenden Salzen ausgewählt ist.

8. Verfahren nach einem der Ansprüche 1-7, **dadurch gekennzeichnet, dass** das Lösungsmittel zum Durchführen der Umsetzung aus der organische Lösungsmittel, vorzugsweise Benzol, Toluol, Xylol, oder chlorierte Lösungsmittel, vorzugsweise Dichlormethan, Dichlorethan, Chloroform, Chlorbenzol, oder Toluol, Xylol oder Acetonitril, Propylencarbonat und verwandte Lösungsmittel umfassenden Gruppe ausgewählt ist.

9. Verfahren nach Anspruch 8, **dadurch gekennzeichnet, dass** das Lösungsmittel zum Durchführen der Umsetzung aus organischen Lösungsmitteln, vorzugsweise Toluol und Xylol, und chlorierten Lösungsmitteln, vorzugsweise aus chlorierten Lösungsmitteln, ausgewählt ist.

10. Verfahren nach einem der Ansprüche 1-7, **dadurch gekennzeichnet, dass** es sich bei dem Katalysator um Lithiumtrifluormethansulfonat handelt und das Lösungsmittel aus organischen Lösungsmitteln, vorzugsweise Toluol und Xylol, und chlorierten Lösungsmitteln, vorzugsweise Dichlormethan, Dichlorethan, Chloroform und/oder Chlorbenzol, ausgewählt ist.

11. Verfahren nach einem der Ansprüche 1-10, **dadurch gekennzeichnet, dass** es sich bei dem entfernbaren Substituenten R um (C₁-C₄)-Alkylcarbonyl, oder gegebenenfalls substituiertes Phenylcarbonyl oder Benzylcarbonyl, vorzugsweise Phenylcarbonyl, Tolylcarbonyl, Xylylcarbonyl; vorzugsweise Acetyl oder p-Chlorphenylcarbonyl, handelt.

12. Verfahren nach einem der Ansprüche 1-11, **dadurch gekennzeichnet, dass** es sich bei dem entfernbaren Substituenten R₁ um Chlor handelt.

## Revendications

1. Méthode de production de 2'-désoxy-5-azacytidine (Décitabine) par la fourniture d'un composé de formule (I) 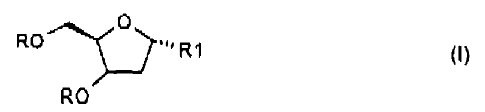 dans laquelle
R est un substituant pouvant être éliminé, connu en soi, préférablement (C₁-C₈)alkylcarbonyle, ou phénylcarbonyle éventuellement substitué, ou benzylcarbonyle éventuellement substitué ;
R₁ est un substituant pouvant être éliminé, préférablement halogène, préférablement chlore, brome, fluor, préférablement chlore, ou un imidate, préférablement l'imidate de trichlorométhyle, ou un dérivé de thioalkyle, préférablement -S-méthyle ;
la fourniture supplémentaire d'une base silylée de formule (II) 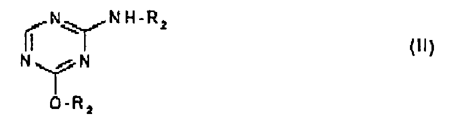 dans laquelle R₂ est un groupement protecteur, préférablement un résidu triméthylsilyle (TMS) ;
la réaction du composé de formule (I) et du composé de formule (II) ensemble dans un solvant anhydre convenable et en présence d'un catalyseur convenable, ce par quoi on obtient le composé de formule (III) 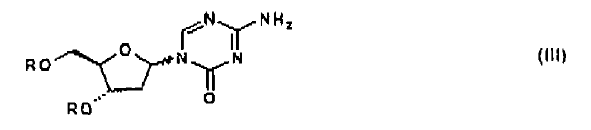 et l'élimination des substituants R afin d'obtenir le composé de 2'-désoxy-5-azacytidine (Décitabine), **caractérisée en ce que** ledit catalyseur est un sel d'un acide sulfonique aliphatique ou un sel d'un acide sulfonique aliphatique fluoré.

2. Méthode de production d'un composé de formule (III) selon la revendication 1, par la fourniture d'un composé de formule (I) 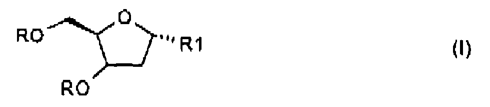 dans laquelle
R est un substituant pouvant être éliminé, connu en soi, préférablement (C₁-C₈)alkylcarbonyle, ou phénylcarbonyle éventuellement substitué, ou benzylcarbonyle éventuellement substitué ;
R₁ est un substituant pouvant être éliminé, préférablement halogène, préférablement chlore, brome, fluor, préférablement chlore, ou un imidate, préférablement l'imidate de trichlorométhyle, ou un dérivé de thioalkyle, préférablement -S-méthyle ;
la fourniture supplémentaire d'une base silylée de formule (II) 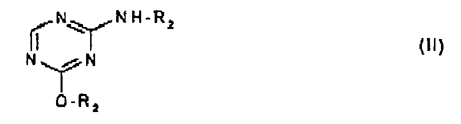 dans laquelle R₂ est un groupement protecteur, préférablement un résidu triméthylsilyle (TMS) ;
la réaction du composé de formule (I) et du composé de formule (II) ensemble dans un solvant anhydre convenable et en présence d'un catalyseur convenable, ce par quoi on obtient le composé de formule (III), **caractérisée en ce que** ledit catalyseur est un sel d'un acide sulfonique aliphatique ou un sel d'un acide sulfonique aliphatique fluoré.

3. Méthode selon la revendication 1 ou 2, **caractérisée en ce que** le catalyseur utilisé dans ladite réaction est un sel d'acide méthylsulfonique ou d'acide éthylsulfonique, ou un sel d'acide trifluorométhanesulfonique, d'acide pentafluoroéthylsulfonique, ou d'acide heptafluoropropylsulfonique.

4. Méthode selon la revendication 3, **caractérisée en ce que** le catalyseur est un sel d'acide méthylsulfonique et/ou les sels d'acide trifluorométhanesulfonique.

5. Méthode selon l'une quelconque des revendications 1-4, **caractérisée en ce que** le catalyseur est un sel alcalin ou un sel alcalino-terreux, préférablement un sel de lithium, sodium, potassium ou magnésium, préférablement un sel de lithium.

6. Méthode selon l'une quelconque des revendications 1-5, **caractérisée en ce que** le catalyseur est le méthylsulfonate de lithium et/ou le trifluorométhanesulfonate de lithium.

7. Méthode selon les revendications 1-4, **caractérisée en ce que** le catalyseur est choisi parmi les sels comprenant les sels de scandium, préférablement Sc(OTf)₃, de zinc, préférablement Zn(OTf)₂, ou de cuivre, préférablement Cu(OTf)₂.

8. Méthode selon l'une quelconque des revendications 1-7, **caractérisée en ce que** le solvant destiné à mettre en oeuvre la réaction est choisi dans le groupe constitué par les solvants organiques, préférablement le benzène, le toluène, le xylène, ou les solvants chlorés, préférablement le dichlorométhane, le dichloroéthane, le chloroforme, le chlorobenzène, ou le toluène, le xylol, ou l'acétonitrile, le carbonate de propylène et les solvants apparentés.

9. Méthode selon la revendication 8, **caractérisée en ce que** le solvant destiné à mettre en oeuvre la réaction est choisi parmi les solvants organiques, préférablement le toluène et le xylène et les solvants chlorés, préférablement parmi les solvants chlorés.

10. Méthode selon l'une quelconque des revendications 1-7, **caractérisée en ce que** le catalyseur est le trifluorométhanesulfonate de lithium, et le solvant est choisi parmi les solvants organiques, préférablement le toluène et le xylène et les solvants chlorés, préférablement le dichlorométhane, le dichloroéthane, le chloroforme et/ou le chlorobenzène.

11. Méthode selon l'une quelconque des revendications 1-10, **caractérisée en ce que** le substituant pouvant être éliminé R est (C₁-C₄)alkylcarbonyle, ou phénylcarbonyle ou benzylcarbonyle éventuellement substitué, préférablement phénylcarbonyle, tolylcarbonyle, xylylcarbonyle ; préférablement acétyl- ou p-chloro-p-phénylcarbonyle.

12. Méthode selon l'une quelconque des revendications 1-11, **caractérisée en ce que** le substituant pouvant être éliminé R₁ est le chlore.
